(19) Europäisches Patentamt
European Patent Office
Office européen des brevets

(11) **EP 3 808 725 A1**

(12) **EUROPÄISCHE PATENTANMELDUNG**

(43) Veröffentlichungstag:
**21.04.2021 Patentblatt 2021/16**

(51) Int Cl.:
***C07C 29/16*** *(2006.01)*      ***C07C 31/04*** *(2006.01)*

(21) Anmeldenummer: **19020578.1**

(22) Anmeldetag: **16.10.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **L'AIR LIQUIDE, SOCIÉTÉ ANONYME POUR L'ÉTUDE ET L'EXPLOITATION DES PROCÉDÉS GEORGES CLAUDE**
**75007 Paris (FR)**

(72) Erfinder:
• **Oelmann, Tobias**
  **61118 Bad Vilbel (DE)**
• **Gronemann, Veronika**
  **61184 Karben (DE)**
• **Hofmann, Heiko**
  **63225 Langen (DE)**
• **Schuhmann, Timm**
  **63067 Offenbach (DE)**

(74) Vertreter: **Dropsch, Holger et al**
**Air Liquide Forschung und Entwicklung GmbH**
**Gwinnerstraße 27-33**
**60388 Frankfurt am Main (DE)**

(54) **VERFAHREN ZUM MEHRSTUFIGEN HERSTELLEN VON METHANOL**

(57)     Es wird ein Verfahren zum Herstellen von Methanol aus Synthesegas mittels mehrstufiger, beispielsweise zweistufiger, heterogen-katalytischer Methanolsynthese vorgeschlagen, wobei der Produktstrom einer Synthesestufe der stromabwärts angeordneten Synthesestufe als Feedstrom aufgegeben oder nach Abtrennen eines Spülstroms als Recyclestrom zu der ersten Synthesestufe zurückgeführt wird. Erfindungsgemäß wird aus dem Synthesegas-Frischgas ein Teilstrom abgetrennt und als Bypassstrom in den zweiten Methanolsynthesereaktor eingeleitet.

Fig. 2

EP 3 808 725 A1

**Beschreibung**

**Gebiet der Erfindung**

Die Erfindung betrifft ein Verfahren zur Herstellung von Methanol durch heterogen-katalysierte Umsetzung von Wasserstoff und Kohlenoxide umfassendem Synthesegas an festen, körnigen Katalysatoren zur Methanolsynthese, die in mehreren, hintereinandergeschalteten Festbettreaktoren angeordnet sind. Die Erfindung betrifft ferner eine Anlage zur Durchführung eines solchen Herstellungsverfahrens.

**Stand der Technik**

Verfahren zur industriellen Herstellung von Methanol durch heterogen-katalytische Umsetzung von Synthesegas, also Gemischen von Wasserstoff und Kohlenoxiden, sind der Fachwelt seit langem bekannt. In Ullmann's Encyclopedia of Industrial Chemistry, Sixth Edition, 1998 Electronic Release, Kapitel "Methanol", Unterkapitel 5.2 "Synthesis" werden verschiedene Grundverfahren zur Herstellung von Methanol durch katalytische Umsetzung von Wasserstoff und Kohlenstoffoxide enthaltendem Synthesegas beschrieben, bei denen solche Reaktoren eingesetzt werden.
Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor (WCR) und dann einem gasgekühlten Reaktor (GCR) zugeführt wird, in welchen das Synthesegas jeweils an einem kupferbasierten Festbett-Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern. Diese Maßnahme wird auch in der deutschen Offenlegungsschrift DE 2934332 A1 und der europäischen Patentanmeldung EP 1016643 A1 gelehrt.
In der wassergekühlten Reaktorstufe wird üblicherweise der Hauptumsatz des Synthesegases ($CO$, $CO_2$, $H_2$) erzielt und der größte Teil der Reaktionswärme abgeführt, während in der gasgekühlten Stufe bei milderen Bedingungen ein dennoch erheblicher Teil des Synthesegases umgesetzt wird.
Bei dem wassergekühlten Reaktor (WCR) handelt es sich üblicherweise um einen Röhrenreaktor mit entsprechenden Rohrplatten, bei dem der Katalysator in die Rohre gefüllt wird, während die Kühlung mittels siedendem Wasser bzw. Dampferzeugung auf der Mantelseite um die Rohre erfolgt. Im gasgekühlten Reaktor (GCR) erfolgt die Kühlung mit dem Einsatzgas, welches durch die Rohre geführt wird und sich auf dem Weg zur ersten Reaktionsstufe (WCR) erwärmt, während der Katalysator um die Rohre gefüllt wird und die Reaktion auf der Mantelseite des GCR stattfindet. Die Reaktionsstufen sind hinsichtlich ihrer Nennweite mit großen bzw. sehr großen Rohrleitungen verbunden; je nach Anlagenkapazität sind Rohrdurchmesser von bis zu 1 m möglich. Dies ist vor allem auf die großen Gasmengen zurückzuführen, die zu der zweiten Stufe zurückgeführt (Recycle-Gas) und dem Frischgas oder Make-up-Gas, also frischem Synthesegas aus der Gaserzeugung, zugemischt werden. Das sich ergebende Gasgemisch aus Rückführungsgas und Frischgas wird nach erfolgter Vorwärmung im GCR der ersten Reaktionsstufe (WCR) zugeführt. Die Rückführungsgasmenge (Recycle-Gas) ist üblicherweise deutlich größer als die Frischgasmenge und ist vom erzielten Umsatz in der Reaktorsektion abhängig. Das Rückführverhältnis RR (RR = R/F) aus Rückführungsgasmenge (R) zu Frischgasmenge (F) liegt oft über 2 und in einigen Fällen sogar über 3,5. Je niedriger der Umsatz von Synthesegas durch die Reaktorsektion per Durchgang ist, um so höher ist das erforderliche Rückführverhältnis RR, um eine hinreichende Ausbeute zu erzielen. Damit erhöht sich die umlaufende Gasmenge entsprechend, was die Belastung der Reaktoren erhöht und größere Rohrnennweiten der verbindenden Rohrleitungen bedarf und auch zu einem höherem Bedarf an Kompressionsenergie (höhere Durchflussmenge und Druckverlust) führt.
Üblicherweise werden in beiden Synthesereaktoren dieselben Methanolsynthesekatalysatoren auf Kupferbasis verwendet, die als feste, körnige Katalysatoren in Festbettreaktoren eingesetzt werden. Bei dem beschriebenen, zweistufigen WCR-GCR-Verfahren wird der wassergekühlte Reaktor typischerweise mit höherer Synthesegas-Eintrittstemperatur betrieben als ein wassergekühlter Reaktor in einem einstufigen Verfahren zur Methanolsynthese, um Dampf mit höherem Druck bereitstellen zu können. Ferner wird dieser Reaktor mit noch nicht abreagiertem Synthesegas beaufschlagt. Aufgrund der hohen Exothermie der Methanolsynthese ist daher eine sehr gute Temperaturkontrolle des Reaktors notwendig, um eine Überhitzung des Katalysators zu vermeiden, die wesentlich zu seiner vorzeitigen Desaktivierung beiträgt. In der DE-Offenlegungsschrift DE 102010008857 A1 wird daher vorgeschlagen, in den beiden Synthesereaktoren Katalysatoren mit unterschiedlicher Aktivität zu verwenden, wobei in dem Reaktor mit den drastischeren Reaktionsbedingungen ein Katalysator mit niedrigerer Aktivität eingesetzt werden soll, der eine geringere Desaktivierungsgeschwindigkeit und somit höhere Langzeitbeständigkeit aufweist.
Nachteiligig bei diesem Verfahren ist es jedoch, dass Katalysatoren mit unterschiedlicher Aktivität bereitgestellt werden müssen, was die für den Anlagenbetrieb bereitzustellende Logistik verkompliziert. Ferner besteht weiterhin Bedarf an einer verbesserten Steuerung der einzelnen Synthesereaktoren, insbesondere im Hinblick auf die dort herrschenden Temperaturen. Lokale Übertemperaturen, sog. "hot spots", sind ein wesentlicher Faktor für eine vorzeitige Desaktivierung der eingesetzten Temperaturen.

**Beschreibung der Erfindung**

Die Aufgabe der vorliegenden Erfindung besteht daher darin, ein Verfahren und eine Anlage anzugeben, das die beschriebenen Nachteile des Stands der Technik nicht aufweist und das es insbesondere ermöglicht, in einem mehrstufigen Verfahren bzw. einer mehrstufigen Anlage zur Methanolsynthese mit mehreren hintereinandergeschalteten Synthesereaktoren eine gleichmäßigere Belastung der in den einzelnen Reaktoren angeordneten Katalysatoren und dort ferner eine verbesserte Temperaturkontrolle zu erreichen.

Diese Aufgabe wird durch ein Verfahren mit den Merkmalen des Anspruchs 1 gelöst. Weitere Ausgestaltungen der Erfindung ergeben sich aus den Unteransprüchen der jeweiligen Kategorie.

Erfindungsgemäßes Verfahren:

Verfahren zum Herstellen von Methanol durch Umsetzen eines Wasserstoff und Kohlenoxide enthaltenden Synthesegaseinsatzstroms, umfassend folgende Verfahrensschritte:

(a) Bereitstellen des Wasserstoff und Kohlenoxide enthaltenden Synthesegaseinsatzstroms, Auftrennen des Synthesegaseinsatzstroms in einen Frischgas-Bypassstrom und in einen Frischgas-Feedstrom,

(b) Zusammenführen und Vermischen des Frischgas-Feedstroms mit einem Wasserstoff und Kohlenoxide enthaltenden Recyclestrom zu einem ersten Reaktorfeedstrom,

(c) Einleiten des ersten Reaktorfeedstroms in einen ersten Methanolsynthesereaktor, enthaltend mindestens eine Katalysatorzone mit einem einen für die Methanolsynthese aktiven, festen, körnigen Katalysator, mindestens teilweises Umsetzen des ersten Reaktorfeedstroms in dem ersten Methanolsynthesereaktor unter Methanolsynthesebedingungen,

(d) Ausleiten eines Methanol und Wasser enthaltenden ersten Reaktorproduktstroms aus dem ersten Methanolsynthesereaktor,

(e) Einleiten des ersten Reaktorproduktstroms als erster Teil eines zweiten Reaktorfeedstroms in einen zweiten Methanolsynthesereaktor, enthaltend mindestens eine Katalysatorzone mit einem einen für die Methanolsynthese aktiven, festen, körnigen Katalysator, mindestens teilweises Umsetzen des ersten Restgasstroms in dem zweiten Methanolsynthesereaktor unter Methanolsynthesebedingungen,

(f) Ausleiten eines Methanol und Wasser enthaltenden zweiten Reaktorproduktstroms aus dem zweiten Methanolsynthesereaktor, Abkühlen des zweiten Reaktorproduktstroms unter seinen Taupunkt und Zuführen des abgekühlten zweiten Reaktorproduktstroms zu einer Phasentrennvorrichtung,

(g) Auftrennen des abgekühlten zweiten Reaktorproduktstroms in der Phasentrennvorrichtung in einen Flüssigproduktstrom und einen Gasproduktstrom, der nicht umgesetzte Synthesegasbestandteile enthält,

(h) Auftrennen des Gasproduktstroms in einen Spülstrom, der aus dem Verfahren ausgeleitet wird, und in den Recyclestrom, der zu Schritt (b) zurückgeführt wird,

(i) Ausleiten des Flüssigproduktstroms aus dem Verfahren als Rohmethanolproduktstrom, **dadurch gekennzeichnet, dass**

(j) der Frischgas-Bypassstrom als zweiter Teil des zweiten Reaktorfeedstroms in den zweiten Methanolsynthesereaktor eingeleitet wird.

Erfindungsgemäße Anlage:

Anlage zum Herstellen von Methanol durch Umsetzen eines Wasserstoff und Kohlenoxide enthaltenden Synthesegaseinsatzstroms, umfassend folgende, miteinander in Fluidverbindung stehende Baugruppen und Bestandteile:

(a) Mittel zum Bereitstellen des Wasserstoff und Kohlenoxide enthaltenden Synthesegaseinsatzstroms, Mittel zum Auftrennen des Synthesegaseinsatzstroms in einen Frischgas-Bypassstrom und in einen Frischgas-Feedstrom,

(b) Mittel zum Zusammenführen und Vermischen des Frischgas-Feedstroms mit einem Wasserstoff und Kohlenoxide enthaltenden Recyclestrom zu einem ersten Reaktorfeedstrom,

(c) einen ersten Methanolsynthesereaktor, enthaltend mindestens eine Katalysatorzone mit einem für die Methanolsynthese aktiven, festen, körnigen Katalysator, Mittel zum Einleiten des ersten Reaktorfeedstroms in den ersten Methanolsynthesereaktor,

(d) Mittel zum Ausleiten eines Methanol und Wasser enthaltenden ersten Reaktorproduktstroms aus dem ersten Methanolsynthesereaktor,

(e) einen zweiten Methanolsynthesereaktor, enthaltend mindestens eine Katalysatorzone mit einem für die Methanolsynthese aktiven, festen, körnigen Katalysator, Mittel zum Einleiten des ersten Reaktorproduktstroms als erster Teil eines zweiten Reaktorfeedstroms in den zweiten Methanolsynthesereaktor,

(f) Mittel zum Ausleiten eines Methanol und Wasser enthaltenden zweiten Reaktorproduktstroms aus dem zweiten Methanolsynthesereaktor, eine Phasentrennvorrichtung, Mittel zum Abkühlen des zweiten Reaktorproduktstroms unter seinen Taupunkt und Mittel zum Zuführen des abgekühlten zweiten Reaktorproduktstroms zu der Phasentrennvorrichtung,

(g) Mittel zum Auftrennen des abgekühlten zweiten Reaktorproduktstroms in der Phasentrennvorrichtung in einen Flüssigproduktstrom und einen Gasproduktstrom, der nicht umgesetzte Synthesegasbestandteile enthält, Mittel zum Ausleiten des Flüssigproduktstroms, Mittel zum Ausleiten des Gasproduktstroms,

(h) Mittel zum Auftrennen des Gasproduktstroms in einen Spülstrom und einen Recyclestrom, Mittel zum Ausleiten des Spülstroms aus dem Verfahren, Mittel zum Zurückführen des Recyclestroms zu Baugruppe (b),

(i) Mittel zum Ausleiten des Flüssigproduktstroms aus dem Verfahren als Rohmethanolproduktstrom, **dadurch gekennzeichnet, dass**

(j) Mittel umfasst werden, die es ermöglichen, den Frischgas-Bypassstrom als zweiter Teil des zweiten Reaktorfeedstroms in den zweiten Methanolsynthesereaktor einzuleiten.

Unter Fluidverbindung zwischen zwei Bereichen des erfindungsgemäßen Reaktors wird dabei jegliche Art von Verbindung verstanden, die es ermöglicht, dass ein Fluid, beispielsweise der Einsatzgasstrom oder der Synthesegasproduktstrom, von dem einen zu dem anderen der beiden Bereiche strömen kann, unbeachtlich etwaiger zwischengeschalteter Bereiche oder Bauteile.

Unter Methanolsynthesebedingungen sind die dem Fachmann an sich bekannten Verfahrensbedingungen, insbesondere von Temperatur, Druck und Verweilzeit, zu verstehen, wie sie oben beispielhaft genannt und detailliert im einschlägigen Schrifttum erörtert werden und bei denen mindestens ein Teilumsatz, bevorzugt allerdings technisch relevante Umsätze der Edukte CO bzw. $CO_2$ und Wasserstoff zum Produkt Methanol erfolgt. Entsprechend wird unter einem für die Methanolsynthese aktiven Katalysator ein Katalysator verstanden, der unter Methanolsynthesebedingungen eben solche Umsätze bewirkt.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile sowie die entsprechenden Durchtrittsöffnungen in Behälterwänden in Betracht ziehen, die ihm aufgrund seines Fachwissens zur Durchführung dieses Verfahrensschrittes notwendig oder sinnvoll erscheinen.

Unter der katalytischen Aktivität, insbesondere im Zusammenhang mit einer unterschiedlichen katalytischen Aktivität beim Vergleich zweier unterschiedlicher Katalysatoren, wird der erzielte Umsatzgrad pro Längeneinheit des Katalysatorbetts von Edukten zu Produkten verstanden. Die Aktivität wird beeinflusst von der chemischen Zusammensetzung, Dotierung, Vergiftung, verfügbaren Oberfläche etc. des Katalysatormaterials, aber auch durch die Geometrie der Katalysatorpartikel und texturellen Parametern des Katalysatorbetts, z. B. dessen Porosität oder Packungsdichte. Aufgrund der Exothermie der betrachteten Reaktionen korreliert eine hohe katalytische Aktivität mit einer hohen Wärmefreisetzung pro Längeneinheit des Katalysatorbetts. Ein weiteres Maß für die Katalysatoraktivität unter festgelegten Methanolsynthesebedingungen stellt der mittels Kondensation aufgefangene Mengenstrom an Flüssigprodukten dar, da die Reaktionsprodukte der Methanolsynthesereaktion gemäß der Umsatzgleichungen

$$CO\ (g) + 2\ H_2\ (g) = CH_3OH\ (l)$$

$$CO_2\ (g) + 3\ H_2\ (g) = CH_3OH\ (l) + H_2O\ (l)$$

unter Umgebungsbedingungen flüssig sind. Demzufolge bezeichnet die Größe "Aktivitätsverlust" die zeitliche Abnahme der Katalysatoraktivität und des Edukt-Umsatzgrades bzw. der Methanol-Ausbeute.

Unter einem ersten bzw. zweiten Methanolsynthesereaktor werden nicht notwendigerweise Einzelreaktoren verstanden, sondern es können auch jeweils Gruppen von Einzelreaktoren von diesem Begriff umfasst werden, die ihrerseits wiederum ein oder mehrere Katalysatorzonen, also mit festem, körnigem Methanolsynthesekatalysator gefüllte Bereiche, beinhalten können. "Erster" bzw. "zweiter" Methanolsynthesereaktor soll dabei lediglich die Durchströmungsreihenfolge der betrachteten Reaktoren andeuten. Der erste bzw. zweite Methanolsynthesereaktor müssen nicht zwangsweise direkt aufeinander folgen, sondern es können weitere, nicht näher betrachtete Methanolsynthesereaktoren zwischengeschaltet sein. Kennzeichnend für den zweiten Methanolsynthesereaktor ist es, dass in diesen erfindungsgemäß der Frischgas-Bypassstrom eingeleitet wird.

Unter einem Katalysatorzyklus wird die Betriebsdauer einer Charge eines Methanolsynthesekatalysators verstanden, beginnend mit der Inbetriebnahme des Methanolsynthesebetriebs des mit der Charge frischen oder regenerierten Methanolsynthesekatalysators befüllten Methanolsynthesereaktors und endend mit der Außerbetriebnahme des Methanolsynthesebetriebs desselben Methanolsynthesereaktors zum Zwecke des Katalysatoraustauschs oder der Durchführung der Katalysatorregenerierung.

Der Erfindung liegt die Erkenntnis zugrunde, dass der Aktivitätsverlust eines Methanolsynthesekatalysators infolge Katalysatordesaktivierung durch verschiedene Hauptkriterien verursacht wird, zu denen insbesondere hohe Temperaturen, die zeitliche Produktionsmenge und die Anlagerung von Katalysatorgiften und der Verlust der aktiven Zentren zählen. In mehrstufigen Verfahren bzw. Anlagen mit hintereinandergeschalteten Reaktorsystemen desaktivieren die Katalysatoren in den einzelnen Synthesereaktoren unterschiedlich schnell. So wird beispielsweise der erste Reaktor in Strömungsrichtung stärker belastet, da er mit reaktiverem Synthesegas beaufschlagt wird, das eine höhere Konzentration der Eduktkomponenten enthält. Hieraus resultieren höhere Maximaltemperaturen im Katalysatorbett und eine anfänglich höhere Produktionsrate, die danach jedoch im Vergleich mit nachgeschalteten Reaktoren rascher absinkt. Wenn trotz Ausnutzung möglicher Betriebsparameteranpassungen die vorgeschriebene Gesamtproduktionskapazität der Anlage nicht mehr erreicht wird, werden die Katalysatoren in den Synthesereaktoren ausgetauscht. Der Katalysator im strömungsmäßig ersten Reaktor zeigt dabei zumeist eine stärkere Desaktivierung als der Katalysator in dem oder den nachgeschalteten Reaktoren. Ein separater Austausch nur des Katalysators im ersten Reaktor ist theoretisch möglich, aber logistisch schwierig, und wird daher vermieden. Auch für den Austausch des Katalysators in nur einem Synthesereaktor muss trotzdem die gesamte Syntheseanlage stillgesetzt werden. Es ist daher wenig attraktiv, nur den Katalysator in einem Synthesereaktor auszutauschen, da ansonsten mit weiteren Stillständen für den Austausch der zunächst weniger desaktivierten Katalysatoren in den nachgeschalteten Reaktoren zu rechnen ist.

Aufgrund der erfindungsgemäßen Synthesegaseinspeisung vor einen nachgeschalteten, beispielsweise den zweiten Synthesereaktor werden alle Reaktionszonen gleichmäßiger belastet, woraus eine gleichmäßigere und damit effizientere Ausnutzung des Katalysators resultiert. Dies führt zu einer möglichen Reduzierung des Rückführverhältnis RR sowie des Reaktorvolumens und ferner zu einer verlängerten Katalysatorlebensdauer, höheren Produktionskapazitäten und damit verbunden zu Kostenreduzierungen für den gesamten Prozess. Erreicht wird dies durch die Einspeisung des reaktiven Synthesegases vor einen nachgeschalteten, beispielsweise den zweiten Methanolsynthesereaktor, der somit von Beginn an mit Synthesegas belastet wird, das eine höhere Konzentration an Eduktkomponenten enthält. Der erste Methanolsynthesereaktor wird aufgrund der Verdünnung des Synthesegases mit Recyclegas weniger belastet. Somit kann der Katalysator in dem nachgeschalteten, beispielsweise im zweiten Methanolsynthesereaktor besser ausgenutzt werden und die Synthese effizienter durchgeführt werden.

Mit zunehmendem Aktivitätsverlust des Katalysators in den Synthesereaktoren kann die Menge an frischem Synthesegas zu einem nachgeschalteten, z. B. zum zweiten Reaktor reduziert werden, um die Verweilzeit des Synthesegases in diesem Synthesereaktor zu erhöhen und somit den zeitlich steigenden Methanol-Ausbeuteverlust zu kompensieren. Ferner kann überraschend mit der erfindungsgemäßen Verschaltung das Rückführverhältnis RR deutlich reduziert werden. Je nach vorgesehener Produktionskapazität und Gaszusammensetzung kann bei Erhalt der Produktionskapazität eine Reduzierung des Rückführverhältnis RR von laut Stand der Technik üblichen Werten zwischen 1,6 und 2,5 auf erfindungsgemäße Werte zwischen 1,0 und 2,0 erfolgen.

**Besondere Ausgestaltungen der Erfindung**

Eine besondere Ausgestaltung des erfindungsgemäßen Verfahrens ist dadurch gekennzeichnet, dass vor dem Einleiten in den zweiten Methanolsynthesereaktor der Frischgas-Bypassstrom mit dem ersten Reaktorproduktstrom zusammengeführt und vermischt wird, wobei der zweite Reaktorfeedstrom erhalten wird. Auf diese Weise wird eine gleichmäßige Reaktantenkonzentration am Eingang des zweiten Methanolsynthesereaktor sichergestellt und die Bildung von Konzentrationssträhnen, die zu einem ungleichmäßigen Reaktorbetrieb führen können, vermieden.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus kontinuierlich oder schrittweise verändert wird. Hierdurch wird ein weiterer Freiheitsgrad erhalten, um einen gleichmäßigen Betrieb der Produktionsanlage sicherzustellen und unerwünschten Exkursionen von den Soll-Betriebsparametern, beispielsweise von Temperaturspitzen in den Katalysatorbetten, entgegenzuwirken.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus kontinuierlich oder schrittweise verringert wird. Nach dieser Ausgestaltung wird beispielsweise mit zunehmendem Aktivitätsverlust des Katalysators in den Synthesereaktoren die Menge an frischem Synthesegas zum zweiten Synthesereaktor reduziert, um die Verweilzeit des Synthesegases in diesem Synthesereaktor zu erhöhen und somit den zeitlich steigenden Methanol-Ausbeuteverlust zu kompensieren. Der verbliebene, nicht zum zweiten Synthesereaktor geführte Frischgas-Anteil wird zusätzlich dem ersten Synthesereaktor zugeführt, so dass die Verweilzeit des Synthesegases im ersten Synthesereaktor sinkt, aber über die Summe beider Reaktoren konstant bleibt.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus in Abhängigkeit von dem Aktivitätsverlust des Katalysators im ersten und/oder zweiten Methanolsynthesereaktor kontinuierlich oder schrittweise verringert wird. Nach dieser Ausgestaltung wird demnach mit zunehmendem Aktivitätsverlust des Katalysators in den Synthesereaktoren die Menge an frischem Synthesegas zum zweiten Synthesereaktor reduziert, um die Verweilzeit des Synthesegases in diesem Synthesereaktor zu erhöhen und somit den zeitlich steigenden Methanol-Ausbeuteverlust zu kompensieren. Der verbliebene, nicht zum zweiten Synthesereaktor geführte Frischgas-Anteil wird zusätzlich dem ersten Synthesereaktor zugeführt, so dass die Verweilzeit des Synthesegases im ersten Synthesereaktor sinkt, aber über die Summe beider Reaktoren konstant bleibt. In dieser Ausgestaltung durchläuft ein größerer Anteil des Frischgases beide Reaktoren, so dass trotz des Aktivitätsverlusts des Katalysators im ersten und/oder zweiten Methanolsynthesereaktor die Methanolausbeute wieder ansteigt oder aufrecht erhalten wird.

Eine Möglichkeit zur Bestimmung der Katalysatoraktivität in den einzelnen Methanolsynthesereaktoren, beispielsweise im ersten oder zweiten Methanolsynthesereaktor, besteht in der Probenahme aus den einzelnen Reaktorproduktströmen über an den Reaktorausgängen angebrachten Probenahmestellen und die Analyse dieser Proben auf dem Fachmann bekannte Weise, so dass der Synthesegas-Umsatz und die Methanolausbeute in den einzelnen Reaktoren bestimmt werden kann.

Eine weitere Ausgestaltung des erfindungsgemäßen Verfahrens ist dadurch gekennzeichnet, dass der Mengenstrom des in den zweiten Methanolsynthesereaktor eingeleiteten Frischgas-Bypassstroms während eines Katalysatorzyklus kontinuierlich oder schrittweise von einem Anfangswert auf einen Endwert verringert wird, wobei der Anfangswert höchstens 40 Vol.-% des Synthesegaseinsatzstroms entspricht. Untersuchungen zeigen, dass mit diesem Anfangswert eine besonders gute Temperaturkontrolle in beiden Reaktoren und eine besonders günstige Methanolausbeute erhalten werden.

Eine weitere Ausgestaltung des erfindungsgemäßen Verfahrens ist dadurch gekennzeichnet, dass der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird. Der gasgekühlte Reaktor erfüllt somit zwei Funktionen, nämlich als Synthesereaktor und als Wärmetauscher bzw. Feedvorwärmer für den WCR. Eine gute Temperaturkontrolle des zweiten Reaktors, wie sie durch die erfindungsgemäße Frischfeedeinspeisung ermöglicht wird, ist daher bei der Ausgestaltung gemäß WCR-GCR-Konzept besonders wichtig.

Eine weitere Ausgestaltung des erfindungsgemäßen Verfahrens ist dadurch gekennzeichnet, dass der zweite Methanolsynthesereaktor als gasgekühlter Reaktor (GCR) ausgestaltet ist, wobei der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird. Diese Ausgestaltung bietet wegen der verbesserten Kühlleistung am Reaktionsgaseintritt Vorteile. Bei der Gleichstromführung vergrößert sich ferner nahezu der Abstand der Temperatur der Kühlrohrwand von dem Taupunkt des Produktgemischs gegenüber der Gegenstromführung. Damit ist auch beim Betrieb des wassergekühlten ersten Reaktors bei tiefen Temperaturen das Risiko einer Kondensation erheblich geringer. Außerdem ergibt sich eine flachere Temperaturkurve über der Reaktorlänge mit geringeren Spitzentemperaturen. Dies ist vorteilhaft für die Laufzeitstabilität des Katalysators.

Eine besondere Ausgestaltung der erfindungsgemäßen Anlage ist dadurch gekennzeichnet, dass sie ferner Mittel zum Zusammenführen und Vermischen des Frischgas-Bypassstroms mit dem ersten Reaktorproduktstrom umfasst. Auf diese Weise wird eine gleichmäßige Reaktantenkonzentration am Eingang des ersten Methanolsynthesereaktor sichergestellt und die Bildung von Konzentrationssträhnen, die zu einem ungleichmäßigen Reaktorbetrieb führen können, vermieden.

Eine weitere vorteilhafte Ausgestaltung der erfindungsgemäßen Anlage ist dadurch gekennzeichnet, dass der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei ferner Mittel umfasst werden, die es gestatten, dass der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird. Der gasgekühlte Reaktor erfüllt somit zwei Funktionen, nämlich als Synthesereaktor und als Wärmetauscher bzw. Feedvorwärmer für den WCR. Eine gute Temperaturkontrolle des zweiten Reaktors, wie sie durch die erfindungsgemäße Frischfeedeinspeisung ermöglicht wird, ist daher bei der Ausgestaltung gemäß WCR-GCR-Konzept besonders wichtig.

Ein weiterer Aspekt der erfindungsgemäßen Anlage ist dadurch gekennzeichnet, dass der zweite Methanolsynthesereaktor als gasgekühlter Reaktor (GCR) ausgestaltet ist und ferner so ausgestattet ist, dass es ermöglicht wird, dass der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird. Diese Ausgestaltung bietet wegen der verbesserten Kühlleistung am Reaktionsgaseintritt Vorteile. Bei der Gleichstromführung vergrößert sich ferner nahezu der Abstand der Temperatur der Kühlrohrwand von dem Taupunkt des Produktgemischs gegenüber der Gegenstromführung. Damit ist auch beim Betrieb des wassergekühlten ersten Reaktors bei tiefen Temperaturen das Risiko einer Kondensation erheblich geringer. Außerdem ergibt sich eine flachere Temperaturkurve über der Reaktorlänge mit geringeren Spitzentemperaturen. Dies ist vorteilhaft für die Laufzeitstabilität des Katalysators.

Ein weiterer Aspekt der erfindungsgemäßen Anlage ist dadurch gekennzeichnet, dass der zweite Methanolsynthesereaktor als gasgekühlter Reaktor (GCR) ausgestaltet ist und einen Reaktormantel und in dessen Inneren eine Vielzahl von Rohren umfasst, wobei der für die Methanolsynthese aktive, feste, körnige Katalysator entweder in den Rohren oder in dem Zwischenraum zwischen der Innenseite des Reaktormantels und der Außenseite der Rohre angeordnet ist und wobei der erste Reaktorfeedstrom als Kühlgas durch den jeweils anderen, nicht Katalysator enthaltenden Bereich geleitet wird. Besonders bevorzugt wird dabei der Katalysator in dem Zwischenraum zwischen der Innenseite des Reaktormantels und der Außenseite der Rohre angeordnet und die Rohre von dem ersten Reaktorfeedstrom als Kühlgas durchströmt.

## Ausführungsbeispiele

Weiterbildungen, Vorteile und Anwendungsmöglichkeiten der Erfindung ergeben sich auch aus der nachfolgenden Beschreibung von Ausführungsbeispielen und den Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Es zeigen:

Fig. 1    eine schematische Darstellung des Verfahrens bzw. der Anlage gemäß einer ersten Ausführungsform der Erfindung mit zwei wassergekühlten Reaktoren,

Fig. 2    eine schematische Darstellung des Verfahrens bzw. der Anlage gemäß einer zweiten Ausführungsform der Erfindung mit einem wassergekühlte Reaktor als erster Reaktor und einem gasgekühlten Reaktor als zweiter Reaktor.

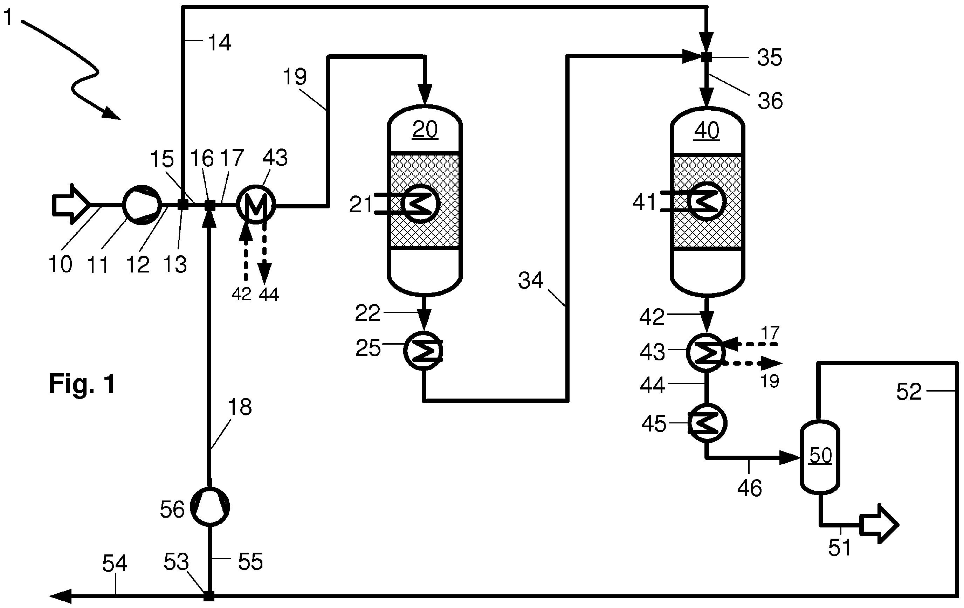

In der in Fig. 1 dargestellten, ersten Ausgestaltung eines Verfahrens 1 bzw. einer Anlage 1 gemäß der Erfindung, die zwei hintereinander geschaltete, wassergekühlte Synthesereaktoren 20, 40 umfasst, wird über Leitung 10 frisches Synthesegas (Frischgas, Make-up-Gas), enthaltend Wasserstoff, Kohlenmonoxid und Kohlendioxid, aus einer nicht dargestellten Synthesegaserzeugungsanlage über Leitung 10 eingeleitet, mittels Verdichter 11 auf Synthesedruck verdichtet und über Leitung 12 zu einer Auftrennvorrichtung 13 geführt, die beispielsweise als T-Rohrstück ausgestaltet sein kann. Aus der Auftrennvorrichtung wird über Leitung 14 ein Teilstrom des Frischgases ausgeleitet und als Frischgas-Bypassstrom zum zweiten Synthesereaktor 40 geführt. Im Verlauf der Leitung 14 kann eine bildlich nicht dargestellte Dosiervorrichtung angeordnet sein, mit deren Hilfe der Mengenstrom des Frischgas-Bypassstroms eingestellt werden kann.

Der verbleibende Anteil des Frischgases wird über Leitung 15 als Frischgas-Feedstrom zu Mischvorrichtung 16 geleitet und in dieser mit einem Recyclestrom zusammengeführt, der über Leitung 18 herangeführt und ebenfalls in die Mischvorrichtung 16 eingeleitet wird.

Die Mischvorrichtung 16 kann, wie auch die nachfolgend genannten Mischvorrichtungen, zum Beispiel als T-Rohrstück oder als statischer Mischer ausgestaltet sein. Das Verhältnis der Mengenströme, die mittels der Leitungen 18 (Recyclestrom) und 15 (Frischgas) zu der Mischvorrichtung 16 geführt werden, entspricht dem Rückführverhältnis RR.

Durch das Zusammenführen und Vermischen des Frischgas-Feedstroms mit dem Recyclestrom wird ein erster Reaktorfeedstrom erhalten, der über Leitung 17 zu Wärmetauscher 43 geführt wird und in diesem im indirekten Wärmetausch gegen den heißen Reaktorproduktstrom aus dem zweiten Synthesereaktor 40 auf die Reaktoreintrittstemperatur aufgeheizt wird. Er wird sodann über Leitung 19 in den ersten Methanolsynthesereaktor 20 eingeleitet.

Im ersten Methanolsynthesereaktor 20, der mindestens eine Katalysatorzone mit einem einen für die Methanolsynthese aktiven, festen, körnigen Katalysator enthält, erfolgt die Teilumsetzung des ersten Reaktorfeedstroms unter Methanolsynthesebedingungen. Im Ausführungsbeispiel der Fig. 1 sind beide Synthesereaktoren 20, 40 wassergekühlt; die jeweiligen in die Reaktoren integrierten Kühlvorrichtungen sind mittels Bezugszeichen 21, 41 angedeutet.

Aus dem ersten Synthesereaktor 20 wird über Leitung 22 ein heißer erster Reaktorproduktstrom ausgeleitet und zu einem optionalen Kühler 25 geführt. Anschließend wird der optional abgekühlte erster Reaktorproduktstrom als erster Teil eines zweiten Reaktorfeedstroms über Leitung 34 zu Mischvorrichtung 35 geführt.

In Mischvorrichtung 35 wird der aufgeheizte erste Restgasstrom mit dem über Leitung 14 herangeführten Frischgas-Bypassstrom als zweiter Teil des zweiten Reaktorfeedstroms zusammengeführt und vermischt. Auch die Mischvorrichtung 35 kann beispielsweise als T-Rohrstück oder als statischer Mischer ausgestaltet sein. Der dabei erhaltene zweite Reaktorfeedstrom wird sodann mittels Leitung 36 in den zweiten Methanolsynthesereaktor 40 eingeleitet, der ebenfalls mindestens eine Katalysatorzone mit einem einen für die Methanolsynthese aktiven, festen, körnigen Katalysator enthält. Im zweiten Methanolsynthesereaktor erfolgt die Teilumsetzung des zweiten Reaktorfeedstroms unter Methanolsynthesebedingungen.

Aus dem zweiten Methanolsynthesereaktor 40 wird über Leitung 42 ein heißer zweiter Reaktorproduktstrom ausgeleitet und zu Wärmetauscher 43 und sodann über Leitung 44 zu Kühler 45 geführt. Im Wärmetauscher 43 erfolgt eine erste Abkühlung des heißen zweiten Reaktorproduktstroms durch indirekten Wärmetausch gegen den über Leitung 17 herangeführten erster Reaktorfeedstrom. Kühler 45 kann beispielsweise als Luftkühler oder als mit Kühlwasser betriebener Kühler ausgestaltet werden. Der unter seinen Taupunkt abgekühlte, zweite Reaktorproduktstrom wird über Leitung 46 in eine Phasentrennvorrichtung 50 eingeleitet und in dieser in einen Flüssigproduktstrom und in einen Gasproduktstrom aufgetrennt. Der Flüssigproduktstrom, der im Wesentlichen Methanol und Wasser enthält, wird über Leitung 51 aus dem Verfahren bzw. aus der Anlage ausleitet und der bildlich nicht dargestellten Rohmethanolaufarbeitung zugeführt. Der Gasproduktstrom, der noch nicht umgesetzte Synthesegasbestandteile enthält, wird über Leitung 52 zu Auftrennvorrichtung 53 geführt.

In Auftrennvorrichtung 53, die die beispielsweise als T-Rohrstück ausgestaltet sein kann, erfolgt die Auftrennung des Gasproduktstroms in einen Spülstrom, der aus dem Verfahren bzw. der Anlage mittels Leitung 54 ausgeleitet wird, und in einen Recyclestrom, der mittels Leitung 55 zu Verdichter 56 geführt wird. Das Ausleiten des Spülstroms über Leitung 54 dient dazu, die Akkumulation von Inertkomponenten wie z. B. Argon oder Methan innerhalb des Synthesekreislaufs zu verhindern. Zum Einstellen des Spülstrom-Mengenstroms kann im Leitungsweg der Leitung 54 ein nicht dargestelltes Dosierventil vorgesehen werden.

Der verdichtete Recyclestrom wird über Leitung 18 aus Verdichter 56 ausgeleitet und in Mischvorrichtung 16 eingeleitet.

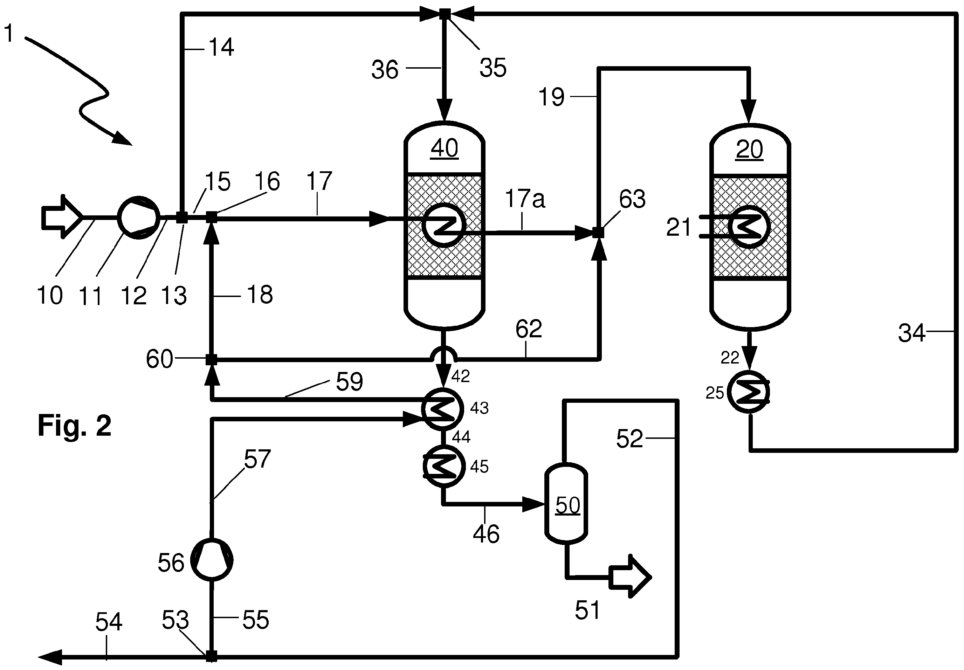

In der in Fig. 2 dargestellten, zweiten Ausgestaltung der Erfindung umfasst das Verfahren 1 bzw. die Anlage 1 einen wassergekühlten Synthesereaktor 20 (WCR) und einen gasgekühlten Synthesereaktor 40 (GCR). Durch gleiche Bezugszeichen gekennzeichnete Zeichnungselemente entsprechen in ihrer Funktion und Beschaffenheit denen, die im Zusammenhang mit Fig. 1 erläutert wurden, sofern im Einzelfall nichts anderes angegeben ist.

Im Vergleich zu der ersten Ausgestaltung ergeben sich für die in Fig. 2 gezeigte Ausgestaltung der Erfindung folgende Unterschiede:

Der erste Reaktorfeedstrom wird über Leitung 17 zunächst zum gasgekühlten Reaktor 40 (GCR), dem zweiten Methanolsynthesereaktor geführt, durchläuft dort die in ihn integrierte Wärmeaustauschvorrichtung (bildlich angedeutet durch den in den Reaktor eingezeichneten Wärmetauscher) und wird dort im indirekten Wärmetausch gegen die heißen Reaktorproduktgase aufgeheizt. Gleichzeitig dient der erste Reaktorfeedstrom somit als Kühlgasstrom im Reaktor 40. Der in Fig. 1 eingezeichnete Wärmetauscher 43 kann im Normalfall entfallen und ist deshalb bildlich in Fig. 2 nicht dargestellt, optional kann er jedoch als Reserve oder zur Realisierung besonderer Betriebszustände, beispielsweise zur Inbetriebnahme der Anlage, vorgesehen werden. Der aufheizte erste Reaktorfeedstrom wird dann über Leitung 17a, Mischvorrichtung 63 und Leitung 19 zum wassergekühlten Reaktor 20 (WCR) als erstem Methanolsynthesereaktor geführt und diesem aufgegeben. Über Leitung 62 kann noch ein Teilstrom des Recyclestroms, das mittels Auftrennvorrichtung 60 abgezweigt wurde, dem ersten Reaktorfeedstrom zur Temperatureinstellung mit Hilfe von Mischvorrichtung 63 zugemischt werden. Die Einstellung des Teilstrom-Mengenstroms erfolgt beispielsweise mit einer nicht gezeigten, in die Leitung 62 integrierten Dosiervorrichtung.

Die Kühlung des aus dem zweiten Methanolsynthesereaktor 40 über Leitung 42 abgeführten, heißen zweiten Reaktorproduktstroms im Wärmetauscher 43 erfolgt im Unterschied zu Fig. 1 mittels des in Leitung 57 herangeführten Recyclestroms als Kühlgas.

Über Leitung 59 wird der aufgeheizte Recyclestrom aus dem Wärmetauscher 43 abgeführt und über Auftrennvorrichtung 60, Leitung 18, Mischvorrichtung 16, Leitung 17, 17a, Mischvorrichtung 63 und Leitung 19 zum ersten Methanolsynthesereaktor 20 zurückgeführt.

**Bezugszeichenliste**

[1]    Verfahren, Anlage

[10]   Leitung
[11]   Verdichter
[12]   Leitung
[13]   Auftrennvorrichtung
[14]   Leitung
[15]   Leitung
[16]   Mischvorrichtung
[17]   Leitung
[18]   Leitung
[19]   Leitung
[20]   erster Methanolsynthesereaktor
[21]   Kühlvorrichtung
[22]   Leitung
[25]   Kühler
[34]   Leitung
[35]   Mischvorrichtung
[36]   Leitung
[40]   zweiter Methanolsynthesereaktor
[41]   Kühlvorrichtung
[42]   Leitung
[43]   Wärmetauscher
[44]   Leitung
[45]   Kühler
[46]   Leitung
[50]   Phasentrennvorrichtung
[51]   Leitung
[52]   Leitung
[53]   Auftrennvorrichtung
[54]   Leitung
[55]   Leitung
[56]   Verdichter
[57]   Leitung
[59]   Leitung
[60]   Auftrennvorrichtung
[62]   Leitung
[63]   Mischvorrichtung

**Patentansprüche**

1.  Verfahren zum Herstellen von Methanol durch Umsetzen eines Wasserstoff und Kohlenoxide enthaltenden Synthesegaseinsatzstroms, umfassend folgende Verfahrensschritte:

(a) Bereitstellen des Wasserstoff und Kohlenoxide enthaltenden Synthesegaseinsatzstroms, Auftrennen des Synthesegaseinsatzstroms in einen Frischgas-Bypassstrom und in einen Frischgas-Feedstrom,
(b) Zusammenführen und Vermischen des Frischgas-Feedstroms mit einem Wasserstoff und Kohlenoxide enthaltenden Recyclestrom zu einem ersten Reaktorfeedstrom,
(c) Einleiten des ersten Reaktorfeedstroms in einen ersten Methanolsynthesereaktor, enthaltend mindestens eine Katalysatorzone mit einem einen für die Methanolsynthese aktiven, festen, körnigen Katalysator, mindestens teilweises Umsetzen des ersten Reaktorfeedstroms in dem ersten Methanolsynthesereaktor unter Methanolsynthesebedingungen,
(d) Ausleiten eines Methanol und Wasser enthaltenden ersten Reaktorproduktstroms aus dem ersten Methanolsynthesereaktor,
(e) Einleiten des ersten Reaktorproduktstroms als erster Teil eines zweiten Reaktorfeedstroms in einen zweiten Methanolsynthesereaktor, enthaltend mindestens eine Katalysatorzone mit einem einen für die Methanolsynthese aktiven, festen, körnigen Katalysator, mindestens teilweises Umsetzen des ersten Restgasstroms in dem zweiten Methanolsynthesereaktor unter Methanolsynthesebedingungen,
(f) Ausleiten eines Methanol und Wasser enthaltenden zweiten Reaktorproduktstroms aus dem zweiten Methanolsynthesereaktor, Abkühlen des zweiten Reaktorproduktstroms unter seinen Taupunkt und Zuführen des abgekühlten zweiten Reaktorproduktstroms zu einer Phasentrennvorrichtung,

(g) Auftrennen des abgekühlten zweiten Reaktorproduktstroms in der Phasentrennvorrichtung in einen Flüssigproduktstrom und einen Gasproduktstrom, der nicht umgesetzte Synthesegasbestandteile enthält,

(h) Auftrennen des Gasproduktstroms in einen Spülstrom, der aus dem Verfahren ausgeleitet wird, und in den Recyclestrom, der zu Schritt (b) zurückgeführt wird,

(i) Ausleiten des Flüssigproduktstroms aus dem Verfahren als Rohmethanolproduktstrom, **dadurch gekennzeichnet, dass**

(j) der Frischgas-Bypassstrom als zweiter Teil des zweiten Reaktorfeedstroms in den zweiten Methanolsynthesereaktor eingeleitet wird.

2. Verfahren nach Anspruch 1, **dadurch gekennzeichnet, dass** vor dem Einleiten in den zweiten Methanolsynthesereaktor der Frischgas-Bypassstrom mit dem ersten Reaktorproduktstrom zusammengeführt und vermischt wird, wobei der zweite Reaktorfeedstrom erhalten wird.

3. Verfahren nach Anspruch 1 oder 2, **dadurch gekennzeichnet, dass** der Mengenstrom des in den zweiten Methanolsynthesereaktor eingeleiteten Frischgas-Bypassstroms während eines Katalysatorzyklus kontinuierlich oder schrittweise verändert wird.

4. Verfahren nach Anspruch 3, **dadurch gekennzeichnet, dass** der Mengenstrom des in den zweiten Methanolsynthesereaktor eingeleiteten Frischgas-Bypassstroms während eines Katalysatorzyklus kontinuierlich oder schrittweise verringert wird.

5. Verfahren nach Anspruch 4, **dadurch gekennzeichnet, dass** der Mengenstrom des in den zweiten Methanolsynthesereaktor eingeleiteten Frischgas-Bypassstroms während eines Katalysatorzyklus in Abhängigkeit von dem Aktivitätsverlust des Katalysators im ersten und/oder zweiten Methanolsynthesereaktor kontinuierlich oder schrittweise verringert wird.

6. Verfahren nach einem der Ansprüche 4 oder 5, **dadurch gekennzeichnet, dass** der Mengenstrom des in den zweiten Methanolsynthesereaktor eingeleiteten Frischgas-Bypassstroms während eines Katalysatorzyklus kontinuierlich oder schrittweise von einem Anfangswert auf einen Endwert verringert wird, wobei der Anfangswert höchstens 40 Vol.-% des Synthesegaseinsatzstroms entspricht.

7. Verfahren nach einem der vorigen Ansprüche, **dadurch gekennzeichnet, dass** der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird.

8. Verfahren nach Anspruch 7, **dadurch gekennzeichnet, dass** der zweite Methanolsynthesereaktor als gasgekühlter Reaktor (GCR) ausgestaltet ist, wobei der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird.

9. Anlage zum Herstellen von Methanol durch Umsetzen eines Wasserstoff und Kohlenoxide enthaltenden Synthesegaseinsatzstroms, umfassend folgende, miteinander in Fluidverbindung stehende Baugruppen und Bestandteile:

(a) Mittel zum Bereitstellen des Wasserstoff und Kohlenoxide enthaltenden Synthesegaseinsatzstroms, Mittel zum Auftrennen des Synthesegaseinsatzstroms in einen Frischgas-Bypassstrom und in einen Frischgas-Feedstrom,

(b) Mittel zum Zusammenführen und Vermischen des Frischgas-Feedstroms mit einem Wasserstoff und Kohlenoxide enthaltenden Recyclestrom zu einem ersten Reaktorfeedstrom,

(c) einen ersten Methanolsynthesereaktor, enthaltend mindestens eine Katalysatorzone mit einem für die Methanolsynthese aktiven, festen, körnigen Katalysator, Mittel zum Einleiten des ersten Reaktorfeedstroms in den ersten Methanolsynthesereaktor,

(d) Mittel zum Ausleiten eines Methanol und Wasser enthaltenden ersten Reaktorproduktstroms aus dem ersten Methanolsynthesereaktor,

(e) einen zweiten Methanolsynthesereaktor, enthaltend mindestens eine Katalysatorzone mit einem für die Methanolsynthese aktiven, festen, körnigen Katalysator, Mittel zum Einleiten des ersten Reaktorproduktstroms als erster Teil eines zweiten Reaktorfeedstroms in den zweiten Methanolsynthesereaktor,

(f) Mittel zum Ausleiten eines Methanol und Wasser enthaltenden zweiten Reaktorproduktstroms aus dem zweiten Methanolsynthesereaktor, eine Phasentrennvorrichtung, Mittel zum Abkühlen des zweiten Reaktorproduktstroms unter seinen Taupunkt und Mittel zum Zuführen des abgekühlten zweiten Reaktorproduktstroms zu der Phasentrennvorrichtung,

(g) Mittel zum Auftrennen des abgekühlten zweiten Reaktorproduktstroms in der Phasentrennvorrichtung in einen Flüssigproduktstrom und einen Gasproduktstrom, der nicht umgesetzte Synthesegasbestandteile enthält, Mittel zum Ausleiten des Flüssigproduktstroms, Mittel zum Ausleiten des Gasproduktstroms,

(h) Mittel zum Auftrennen des Gasproduktstroms in einen Spülstrom und einen Recyclestrom, Mittel zum Ausleiten des Spülstroms aus dem Verfahren, Mittel zum Zurückführen des Recyclestroms zu Baugruppe (b),

(i) Mittel zum Ausleiten des Flüssigproduktstroms aus dem Verfahren als Rohmethanolproduktstrom, **dadurch gekennzeichnet, dass**

(j) Mittel umfasst werden, die es ermöglichen, den Frischgas-Bypassstrom als zweiter Teil des zweiten Reaktorfeedstroms in den zweiten Methanolsynthesereaktor einzuleiten.

10. Anlage nach Anspruch 9, ferner umfassend Mittel zum Zusammenführen und Vermischen des Frischgas-Bypassstroms mit dem ersten Reaktorproduktstrom.

11. Anlage nach einem der vorigen Ansprüche, **dadurch gekennzeichnet, dass** der erste Methanolsynthesereaktor als wassergekühlter Reaktor (WCR) und der zweite Methanolsynthesereaktor als wassergekühlter Reaktor (WCR) oder gasgekühlter Reaktor (GCR) ausgestaltet ist, wobei ferner Mittel umfasst werden, die es gestatten, dass der erste Reaktorfeedstrom vor dem Einleiten in den ersten, wassergekühlten Methanolsynthesereaktor als Kühlgas durch den zweiten, gasgekühlten Methanolsynthesereaktor geführt wird und dabei im indirekten Wärmetausch gegen den zweiten Reaktorproduktstrom erhitzt wird.

12. Anlage nach Anspruch 11, **dadurch gekennzeichnet, dass** der zweite Methanolsynthesereaktor als gasgekühlter Reaktor (GCR) ausgestaltet ist und ferner so ausgestattet ist, dass es ermöglicht wird, dass der erste Reaktorfeedstrom im Gleichstrom zu dem zweiten Reaktorproduktstrom durch den zweiten, gasgekühlten Methanolsynthesereaktor geführt wird und dabei im indirekten Wärmetausch gegen den zweiten Reaktorproduktstrom erhitzt wird.

13. Anlage nach Anspruch 11 oder 12, **dadurch gekennzeichnet, dass** der zweite Methanolsynthesereaktor als gasgekühlter Reaktor (GCR) ausgestaltet ist und einen Reaktormantel und in dessen Inneren eine Vielzahl von Rohren umfasst, wobei der für die Methanolsynthese aktive, feste, körnige Katalysator entweder in den Rohren oder in dem Zwischenraum zwischen der Innenseite des Reaktormantels und der Außenseite der Rohre angeordnet ist und wobei der erste Reaktorfeedstrom als Kühlgas durch den jeweils anderen, nicht Katalysator enthaltenden Bereich geleitet wird.

Fig. 1

EP 3 808 725 A1

Fig. 2

**Europäisches Patentamt**
**European Patent Office**
**Office européen des brevets**

# EUROPÄISCHER RECHERCHENBERICHT

Nummer der Anmeldung

EP 19 02 0578

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2006/018610 A1 (DAVY PROCESS TECHN LTD [GB]; EARLY SIMON ROBERT [GB]) 23. Februar 2006 (2006-02-23) * Anspruch 1; Abbildungen 1,2 * | 1-13 | INV. C07C29/16 C07C31/04 |
| | ----- | | |
| A,D | EP 0 790 226 A1 (METALLGESELLSCHAFT AG [DE]) 20. August 1997 (1997-08-20) * das ganze Dokument * | 1-13 | |
| | ----- | | |
| A | WO 2017/121981 A1 (JOHNSON MATTHEY DAVY TECHNOLOGIES LTD [GB]) 20. Juli 2017 (2017-07-20) * das ganze Dokument * | 1-13 | |
| | ----- | | |

RECHERCHIERTE SACHGEBIETE (IPC)

C07C

Der vorliegende Recherchenbericht wurde für alle Patentansprüche erstellt

| Recherchenort | Abschlußdatum der Recherche | Prüfer |
|---|---|---|
| Den Haag | 1. April 2020 | Fritz, Martin |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 19 02 0578

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-04-2020

| Im Recherchenbericht angeführtes Patentdokument | Datum der Veröffentlichung | Mitglied(er) der Patentfamilie | Datum der Veröffentlichung |
|---|---|---|---|
| WO 2006018610 A1 | 23-02-2006 | AR 051075 A1 | 20-12-2006 |
| | | AT 455592 T | 15-02-2010 |
| | | AU 2005273687 A1 | 23-02-2006 |
| | | BR PI0514433 A | 10-06-2008 |
| | | CN 1989092 A | 27-06-2007 |
| | | EA 200700326 A1 | 29-06-2007 |
| | | EG 24503 A | 18-08-2009 |
| | | EP 1778610 A1 | 02-05-2007 |
| | | MY 141554 A | 14-05-2010 |
| | | NO 338659 B1 | 26-09-2016 |
| | | PL 1778610 T3 | 30-06-2010 |
| | | US 2007225385 A1 | 27-09-2007 |
| | | WO 2006018610 A1 | 23-02-2006 |
| | | ZA 200701478 B | 30-04-2008 |
| EP 0790226 A1 | 20-08-1997 | CA 2197574 A1 | 16-08-1997 |
| | | CN 1163255 A | 29-10-1997 |
| | | DE 19605572 A1 | 21-08-1997 |
| | | DK 0790226 T3 | 07-08-2000 |
| | | EP 0790226 A1 | 20-08-1997 |
| | | IN 186506 B | 22-09-2001 |
| | | US 5827901 A | 27-10-1998 |
| WO 2017121981 A1 | 20-07-2017 | AU 2016386959 A1 | 14-06-2018 |
| | | CA 3006751 A1 | 20-07-2017 |
| | | CL 2018001866 A1 | 23-11-2018 |
| | | CN 108463449 A | 28-08-2018 |
| | | EA 201891603 A1 | 28-12-2018 |
| | | EP 3402772 A1 | 21-11-2018 |
| | | GB 2550994 A | 06-12-2017 |
| | | US 2019016655 A1 | 17-01-2019 |
| | | WO 2017121981 A1 | 20-07-2017 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0790226 B1
- DE 2934332 A1
- EP 1016643 A1
- DE 102010008857 A1

**In der Beschreibung aufgeführte Nicht-Patentliteratur**

- Methanol'', Unterkapitel 5.2 ''Synthesis. Ullmann's Encyclopedia of Industrial Chemistry. 1998